# EUROPEAN PATENT SPECIFICATION

(11) **EP 1 875 918 B1**
(45) Date of publication and mention of the grant of the patent: **18.09.2013**
(21) Application number: 06386019.1
(22) Date of filing: 03.07.2006
(51) Int. Cl.: A61K 33/02, A61K 9/28, A61P 1/16

(54) **New use of ammonium chloride for the therapy of total or partial hepatic failure and necrosis**
Neue Verwendung von Ammonium Chlorid für die Behandlung von totalen oder partiellen Leberschwäche und Nekrose
Nouvelle utilisation du chlorure d'ammonium pour le traitement de la défaillance hépatique totale ou partielle et de la nécrose

(43) Date of publication of application: 09.01.2008
(73) Proprietor: RAINBOW PHARMACEUTICAL Sa, 2121 Luxembourg (LU)
(72) Inventor: Kiassos, Diamantis, 185 36 Piraeus (GR)
(74) Representative: Long, Giorgio

(56) References cited:
- EP-A1- 0 167 191
- GR-A- 2002 100 405
- GR-B1- 1 003 980
- US-A- 4 346 082
- WAN YUNG SHIN ET AL: "AMMONIUM CHLORIDE TOLERANCE TESTS IN RABBITS INTRAVENOUSELY ADMINISTERED WITH MULTIPLE DOSES OF CARBON TETRACHLORIDE" GORYE DAIHAN-YO JABJI - KOREA UNIVERSITY MEDICAL JOURNAL, KORYO TAEHAKKYO UIGWA TACHAK, SEOUL, KR, vol. 9, no. 1, 1972, pages 113-123, XP001051456 ISSN: 0378-648X
- SUPERTI F ET AL: "THE EFFECT OF LIPOPHILIC AMINES ON THE GROWTH OF HEPATITIS A VIRUS IN FRP-3 CELLS" ARCHIVES OF VIROLOGY, NEW YORK, NY, US, vol. 96, no. 3-4, 1987, pages 289-296, XP009078242 ISSN: 0304-8608

## Description

The present invention constitutes the use of ammonium chloride in a method for the pharmaceutical treatment of hepatic failure and necrosis, which is caused by various toxic agents and viral infections.

There are numerous pathologic and toxic conditions that lead to a degenerative malfunction of the liver. Among these are hepatitis C (1/3 of the infected present hepatic cirrhosis), chronic hepatitis B & D, autoimmune hepatitis, non-alcoholic steatohepatitis, colangity sclerosante, Wilson's disease and liver Carcinoma.

As regards toxic conditions, the most common and relevant is the one that occurs due to abuse of alcohol. Other toxic conditions are due to drugs and chemical hepatoxic agents that may lead to hepatic necrosis, such as the poison of certain mushrooms (AMANITA FALLOIDES).

When a big part of the hepatic parenchyma gets necrotized, the hepatic function decreases and may quickly lead to the death of the patient.

Today there are no special treatments able to solve the hepatic necrosis problem, and the patients that suffer from these degenerative diseases have a limited prognosis in relation to the survival average.

NH₄Cl is a water-soluble inorganic salt, which is used for the treatment of hypochloremic metabolic alkalosis. It is also used for diagnostic purposes. It is administered up to a concentration point of 15N, and through the isotopic ammonia & urea analysis of the urine of the patient, we are able to evaluate the hepatic function.

In the Greek patent no. 1003980, the researcher describes the way, in which NH₄Cl decreases the levels of billirubin in patients with severe hepatic malfunctions and hepatic encephalopathy, which is caused by the increase of the billirubin levels. These levels may be decreased with the use of NH₄Cl.

Working on an experimental study on Winstar rats, which suffered from acute hepatitis caused with the known method of TAA administration, the researcher found out that the histological samples of the liver of the rats that had been administered NH₄Cl were much better than the ones of the placebo treatment. He noticed a partial or complete regeneration of the hepatic parenchyma. Furthermore, he noticed a decrease of the TNF-a (tumoral factor necrosis) levels, while the levels of IL-6 were increased.

The levels of hyaluronic acid at patients with high-grade hepatic damage are high, but they appeared decreased as regards the Winstar rats. Furthermore, additional biochemical factors appeared improved.

The most common side effect of the NH₄Cl administration is the metabolic oxidation, which is due to the increase chlorides and the irritation of the gastric mucous. The first side effect may be confronted with the administration of the medication for 3 days and an interval of 4 days. This cycle may be repeated several times until the completion of the treatment (21 cycles, which complies with the natural time of regeneration of liver after a partial hepatectomy in patients).

The irritation of the gastric mucous may be confronted with the enclosure of NH₄Cl capsules or water-soluble acid resistant envelopes.

The dosage varies between 2-5gr. in the form of granules or microcapsules with polymer compounds for the protection of the stomach.

In order to have a more constant blood concentration of NH₄Cl after administration, the tablets, capsules or granules can be filled with a polymer or the matrix of a pharmaceutical form can be made of excipient that slows the release of active ingredient in the gastro-intenstinal lumen.

## Claims

1. Ammonium chloride for use as a medicament which improves or resolves the hepatic failure due to necrosis of hepatic cell, with various degrees, caused by various reasons, in a scheme of treatment wherein ammonium chloride is administered in a dosage of between 2g to 5g per day for 3 days with an interval of 4 days, for a time period of 1-6 months and 3-4 months for patients with partial necrosis.

2. Ammonium chloride for use according to claim 1, wherein the said NH4Cl medicament is in a pharmaceutical form that slows the release of active ingredient in the gastro-intestinal lumen, or in the forum of tablets or capsules coated with polymer compounds for the protection of the stomach or in the form of granules or microcapsules with polymer compounds for the protection of the stomach, or in a ready to use suspension.

3. Ammonium chloride for use according to any one of claims 1 or 2, wherein said ammonium chloride is associated with interferons and antiviral for the improved treatment of viral hepatitis.

## Patentansprüche

1. Ammoniumchlorid zur Verwendung als ein Medikament, welches Leberversagen aufgrund von Leberzellnekrose mit unterschiedlichen Ausmaßen, durch verschiedene Ursachen verursacht, verbessert oder abheilt, in einem Behandlungsschema, wobei Ammoniumchlorid in einer Dosis von zwischen 2g bis 5g pro Tag, für drei Tage, mit einem Interval von 4 Tagen, über einen Zeitraum von 1-6 Monate und 3-4 Monate für Patienten mit partieller Nekrose verabreicht wird.

2. Ammoniumchlorid zur Verwendung gemäß Anspruch 1, wobei das NH4Cl-Medikament in einer pharmazeutischen Form ist, welche die Freisetzung des aktiven Inhaltsstoffs in das gastrointestinale Lumen verlangsamt, oder in der Form von Tabletten oder Kapseln, welche zum Schutz des Magens mit Polymerverbindungen beschichtet sind oder in der Form von Granulat oder Microkapseln mit Polymerverbindungen zum Schutz des Magens, oder in einer gebrauchsfertigen Suspension.

3. Ammoniumchlorid zur Verwendung gemäß einem der Ansprüche 1 oder 2, wobei das Ammoniumchlorid zur verbesserten Behandlung von viraler Hepatitis mit Interferonen und Antiviral assoziiert ist.

## Revendications

1. Chlorure d'ammonium destiné à être utilisé comme médicament qui améliore ou résout l'insuffisance hépatique due à une nécrose de cellule hépatique, avec différents degrés, causée par différentes raisons, dans un schéma de traitement où le chlorure d'ammonium est administré en une posologie entre 2g et 5 g par jour pendant 3 jours avec un intervalle de 4 jours, pendant une durée de 1-6 mois et 3-4 mois pour les patients ayant une nécrose partielle.

2. Chlorure d'ammonium destiné à être utilisé selon la revendication 1 où ledit médicament NH4CI est dans une forme pharmaceutique qui ralentit la libération d'ingrédient actif dans la lumière gastro-intestinale, ou sous forme de comprimés ou capsules enrobés de composés polymères pour la protection de l'estomac ou sous forme de granulés ou de microcapsules avec des composés polymères pour la protection de l'estomac, ou dans une suspension prête à l'emploi.

3. Chlorure d'ammonium destiné à être utilisé selon l'une quelconque des revendications 1 ou 2 où ledit chlorure d'ammonium est associé avec des interférons et un antiviral pour le traitement amélioré de l'hépatite virale.
